Europäisches Patentamt

European Patent Office   (11) Publication number:   **0 041 254**

Office européen des brevets   **A1**

## (12) EUROPEAN PATENT APPLICATION

(21) Application number: **81104135.9**

(22) Date of filing: **29.05.81**

(51) Int. Cl.³: **A 61 N 1/04**

(30) Priority: **30.05.80 DE 3020586**

(43) Date of publication of application:
**09.12.81 Bulletin 81/49**

(84) Designated Contracting States:
**AT BE CH FR GB IT LI LU NL SE**

(71) Applicant: **Bisping, Hans-Jürgen, Dipl.-Ing.**
**Tittardshang 12**
**D-5100 Aachen-Laurensberg(DE)**

(72) Inventor: **Bisping, Hans-Jürgen, Dipl.-Ing.**
**Tittardshang 12**
**D-5100 Aachen-Laurensberg(DE)**

(74) Representative: **Finck, Dieter K.L. Schiff; Dr. A. v. Füner**
**Dipl. Ing. P. Strehl et al,**
**Dr. U. Schübel-Hopf Dipl. Ing. D. Ebbinghaus; Dr. Ing. D.**
**Finck Patentanwälte Mariahilfplatz 2 & 3**
**D-8000 München 90(DE)**

(54) Implantable pacemaker leads.

(57) An implantable lead adapted for use in a pacing system, having an insulated conductor for conducting electrical signals from about its proximal end to its distal end, a stimulating electrode at about said distal end, and fixation means substantially adjacent to said electrode for fixing said distal end to a patient's heart wall, characterized by comprising means for detachably connecting said fixation means whereby said lead conductor can be removed from said patient while said fixation means stays fixed to said patient's heart wall.

FIG. 2

EP 0 041 254 A1

0041254
EPA-20637

## IMPLANTABLE PACEMAKER LEADS

Background Of The Invention

The invention incorporates an implantable lead which forms the electrical contact between a pacemaker and excitable cardiac tissue, and which consists of an electrical conductor having a conductor wire and an insulated portion, and having at its distal end an electrode tip that is fitted with a fixation mechanism.

Various ways are known of manufacturing electrode tips that give stable fixation with the heart. An electrode configuration is described in U.S. Patent No. 4,026,303 which consists of a closed spiral that acts as both an anchoring mechanism and a means of transferring electrical signals. In the course of time tissue ingrowth into the open groves of the electrode takes place, thereby firmly anchoring the tip. An electrode is also known that is anchored by means of tissue ingrowth around synthetic prongs on the electrode tip. An endocardial electrode is described in the patent literature in which contact surfaces are designed and arranged in such a way that they form boundaries of at least a hollow body or a cavity. In the above mentioned configurations secure fixation is achieved by means of tissue ingrowth.

In certain cases such as infection of the pacing system or stimulation sensing problems at the elctrode tip, it can become necessary to remove an implanted electrode. In the above-described cases removal of an electrode is not without risk and can sometimes be absolutely impossible. In addition to the above-mentioned fixation mechanisms there exist a whole range of fixation possibilities which are embodied in various electrode tip configurations. The various types of leads, however, mostly differ only with regard to their tip, the lead conductor being in many cases identical. Because of the fact that the various makes of pacemakers also have different lead connection systems, and therefore often need adapters, there is an unnecessarily large number of leads on the market - making it impossible to lower manufacturing and stock control costs.

It is therefore the aim of this invention to devise a lead that avoids the above-mentioned problems, is easy and reliable in fixation, and which can also be removed from the heart after any given period of time. In addition it is a modular system that is easy to manufacture and store, and which under certain circumstances also gives the physician the possibility of using any one of various electrode tips, depending on the particular case.

## Summary of the Invention

The solution to these problems lies in the invention of a lead in which the fixation mechanism of an electrode tip, in whatsoever configuration it may be, or any part that is constructionally connected to it, therefore normally speaking the complete electrode tip, is detachable. In the case of it being necessary to remove the lead it is usually sufficient to withdraw the conductor without the electrode tip or the fixation mechanism. The fixation mechanism, which is partially securely fixed because of tissue ingrowth, and which cannnot

V-100

0041254

-3-

be removed without major surgical intervention, will not normally present a problem for the patient if it remains in the heart.

In what way and in what position the connection between the conductor and the fixation mechanism is made is dependent on the constructional properties of the lead. Where the ingrown electrode tip is capable of accepting the torque, a screw connection between the conductor and the electrode tip, or between the electrode tip and the fixation mechanism, is a possible embodiment. By applying torque to the proximal end of the conductor it can be separated from the electrode tip or fixation mechanism. With spiralshaped conductors it is advisable to introduce a stylet into the conductor lumen during the procedure, in order to prevent loop forming. It is also an embodiment, by use of a suitable stylet, to achieve a frictional connection with the distal end of the conductor and to transfer the torque solely by means of the stylet, which must be rotated. If, in contrast, the lead has a conductor that does not have a lumen for the introduction of a stylet, or if the conductor itself is not capable of transferring the torque, it is suggested that a guide tube be slid over the lead. In accordance with the invention the distal end of the guide tube is fitted with slots or grooves, which fit into a corresponding part on the distal end of the lead, thereby achieving frictional connection between the parts. The required torque can then be transferred from the guide tube to the electrode tip.

If, by comparision, the electrode tip is axially anchored by the tissue ingrowth but can be rotated about its axis, the above described stylet can also be used to loosen a screw connection.

In order to achieve this the distal end of the stylet is, for example, flattened, so that it fits into a groove in the electrode tip. The required torque is then transferred via the conductor.

If, for example, the lead to be removed has a porous textile cover into which tissue has grown, the following solutions may be utilized:

The textile cover can be fitted with a sleeve, the inside of which has a screw thread that is screwed onto the lead or electrode tip. In such a case, after screwing off the lead only the sleeve with the textile cover remains in the heart. It is also possible to connect the detachable parts by means of a press-fit with precisely defined static and sliding friction. By applying the required force to the conductor, i.e., greater than 100 pounds, the connected parts can be separated. In order to avoid applying too much pressure on the electrode bed it is also possible to first push a guide tube over the conductor, of the type that is sometimes used during introduction of electrodes, until its distal end encounters the house with fixation mechanism. By pulling on the proximal end of the conductor it is possible to surmount greater frictional and adherence resistances.

Connections are also favorable that are released simply by making use of an attachment such as a thin nylon thread, that is inside a helical conductor wire. If necessary, contact clips can be used in order to ensure that electrical contact is maintained.

Manufacture and storage of leads is much simplified by making use of a modular system, because the conductor can be fitted with any one of various electrode tips. This can be done either by the manufacturer of the user.

The invention also includes the use of radiopaque markings on both the detachable electrode tip and on the proximal part of the conductor. This makes radiological control possible at all times.

It is also suggested that the connection between the electrode tip and the conductor, or between the fixation mechanism and the conductor, is a male-female configuration (screw or press-fit).. It is of great advantage to have the section remaining in the heart designed as the female portion, and the conductor to comprise the male connection. This configuration has the advantage that the dimensions of that part of the electrode that remains in the heart can be kept to a minimum.

## Brief Description of the Drawings

For a further description of the invention we refer to the drawings, in which four possible versions are shown in a simplified manner.
They are:

Fig. 1 is a partial longitudinal cross-section through a lead with an electrode tip that has a screw connection;

Fig. 2 is a partial longitudinal cross-section of the same lead as shown in Fig. 1, with a press fitting between the detachable parts;

Fig. 3 is a partial longitudinal cross-section of the same lead as shown in Fig. 2 with a screw connection; and

Fig. 4 is a schematic partial longitudinal cross-section of a lead with a blocking mechanism.


## Description of the Preferred Embodiments

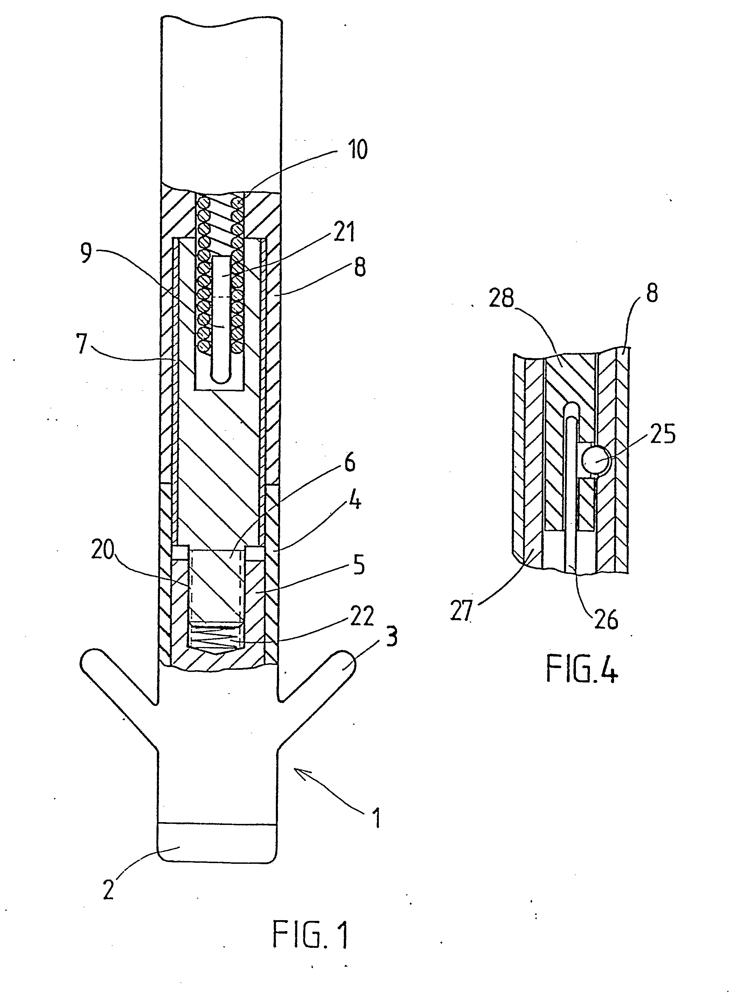

In Figs. 1 to 3, 1 indicates an electrode tip with a stimulation surface area 2 at it distal end. The electrode tip 1 is also fitted with an anchor shaped fixation mechanism 3, which can be anchored in the heart tissue and will then be subject to tissue ingrowth. On the side opposite the stimulation surface area 2 of the electrode tip an insulation sleeve 4 is fitted, inside which a second sleeve 5 is found, this second sleeve being fitted to the stimulation surface area 2 and also having an internal screw thread 20. A connector 6 is screwed into the sleeve 5 and connected to a spiral shaped conductor 10, conductor 10 and part of the connector 6 having an insulation tube 8 around them. A teflon sleeve 7 is fitted between insulation tube 8 and insulation sleeve 4 and connector 6. A contact clip 22 is fitted between connector 6 and sleeve 5 in order to improve the electrical contact. The conductor is fixed into the connector 6 by means of a pin 9 which has a profiled section 21 in the form of a groove into which a stylet 22, for example, can grip.

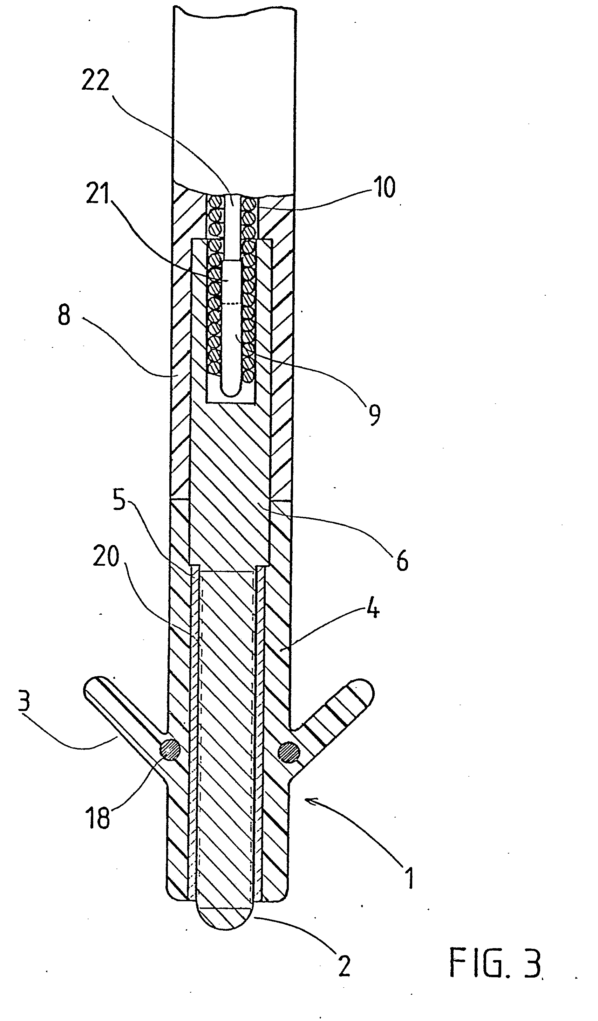

The embodiment of Fig. 3 differs from Fig. 1 in that the fixation mechanism 3 together with the insulation sleeve 4 and sleeve 5 can be unscrewed form the connector. This connector passes through sleeve 5 and froms the stimulation surface area 2 at its distal end. For better radiological control the detachable part, in this case the fixation mechanism 3 comprised of synthetic anchors, has radiopague markings in the form of a wire ring 18.

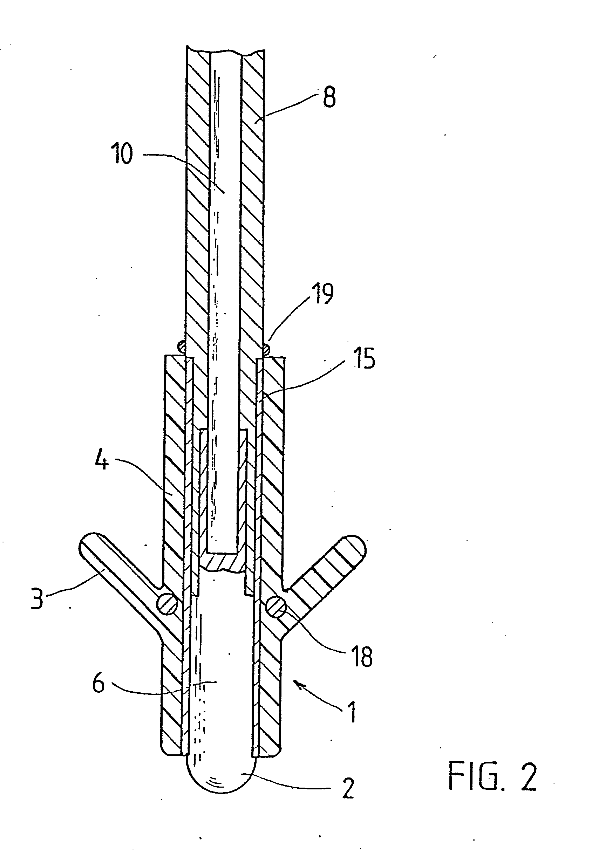

The version shown in Fig. 2 has, in addition to the insulation sleeve 4, a teflon sleeve 15. These two sleeves are slid over the insulation tube 8 and have a press-fit connection. In order to prevent the insulation sleeve from sliding too far the insulation tube 8 is fitted with a ring-shaped stop 19. The connector 6, which here also forms the stimulation surface area 2 at its dital end, is also press-fitted between the insulation tube 8 and the conductor 10 inside teflon sleeve 15 and insulation sleeve 4.

The blocking mechanism shown in Fig. 4 is fitted with a ball 25 located partially in external sleeve 27. Sleeve 27 is inside insulation sleeve 8, and is connected to the conductor. The ball can fall into a cavity in the internal sleeve 28. The internal sleeve 28 is connected to the electrode tip and the fixation mechanism in any convenient manner. The ball 25 is shown in its blocking position, i.e. in the cavity of the external sleeve 27, and is normally kept in place by a wire so that the electrode tip is fixed to the conductor. After removal of the wire 26, the ball 25 is freed so that the electrode tip with the fixation mechanism can be removed from the conductor.

Claims

1.  An implantable lead which maintains electrical contact between a pacemaker and excitable cardiac tissue, and comprising a conductor portion having a conductor wire and insulation around same, and an electrode tip and a fixation mechanism at about its distal end, at least said fixation mechanism being detachable relative to said conductor portion.

2.  The lead as described in claim 1, said lead having a combined fixation mechanism and electrode tip portion that can be screwed off from said conductor portion.

3.  The lead as described in claim 1or 2, having a part distally connected to said conductor which has a profiled section adapted to facilitate receipt of a stylet, whereby said conductor portion can be unscrewed from said fixation mechanism and withdrawn.

4.  The lead as described in claim 1, comprising press-fit means for providing a press-fit connection between said fixation mechanism and said electrode tip.

5.  The lead as described in claim 1, said lead having between said electrode tip and said conductor a blocking mechanism adapted to be released by remote control at the proximal end of said conductor.

6.    The lead as described in one of the previous claims, said lead having radiopaque markings mounted in said fixation mechanism.

7.    The lead as described in claim 1, comprising means for providing a detachable connection between said fixation mechanism and said electrode tip.

8.    The lead as described in claim 1, comprising means for providing a detachable connection between said electrode tip and said conductor portion.

9.    An implantable lead adapted for use in a pacing system, having an insulated conductor for conducting electrical signals from about its proximal end to its distal end, a stimulating electrode at about said distal end, and fixation means substantially adjacent to said electrode for fixing said distal end to a patient's heart wall, characterized by comprising means for detachably connecting said fixation means whereby said lead conductor can be removed from said patient while said fixation means stays fixed to said patient's heart wall.

FIG. 1

FIG. 4

FIG. 2

FIG. 3

**0041254**

## European Patent Office

### EUROPEAN SEARCH REPORT

Application number
EP 81 10 4135

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | **A 61 N 1/04** |
| X | <u>EP - A - 0 005 106</u> (SYNTHELABO)<br><br>* page 5, line 24 to page 6, line 7 * | 1,4,7, 9 | |
| | <u>US - A - 3 835 864</u> (RASOR)<br><br>* column 4, lines 31-42; column 5, lines 13-22; column 7, lines 54-67; column 9, line 44 to column 10, line 16 * | 1,2,8 | |
| | <u>DE - A - 2 053 919</u> (SCHALDACH)<br><br>* page 3, lines 1-6 * | 1,8 | **TECHNICAL FIELDS SEARCHED (Int. Cl.³)**<br><br>A 61 N 1/04 |
| | <u>US - A - 4 136 701</u> (BARTON)<br><br>* column 2, lines 52-65; column 3, lines 59-69 column 4, lines 8-24; figure 5 * | 2,3 | |
| | <u>FR - A - 2 319 384</u> (NEDTRONIC)<br><br>* page 4, lines 14-19 * | 6 | |
| P,E | <u>NL - A - 79 09 050</u> (URCULLU)<br><br>* page 1, lines 20-39; figure 2 * | 1,4,7, 9 | |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 01-09-1981 | SIMON |

EPO Form 1503.1   06.78